# EUROPEAN PATENT APPLICATION

(11) **EP 4 358 096 A1**
(43) Date of publication of application: **24.04.2024**
(21) Application number: 23186130.3
(22) Date of filing: 18.07.2023
(51) Int. Cl.: G16H 50/20, G16H 50/30, G16H 50/70, G16H 30/40, G16H 10/40, G16H 10/60, A61B 5/00, A61B 6/03, G06T 7/00, G06V 10/82

(54) **DISEASE PREDICTION METHOD AND APPARATUS**

(30) Priority: 18.10.2022 KR 20220134461; 24.11.2022 KR 20220159560
(71) Applicant: Medicalip Co., Ltd., Gangwon-do 24341 (KR)
(72) Inventor: Park, Sang Joon, Seoul (KR); Kim, Jong Min, Yongin-si (KR); Chung, Han Jae, Seoul (KR)
(74) Representative: Patentanwälte Ruff, Wilhelm, Beier, Dauster & Partner mbB

(57) **Abstract**

Provided is a disease prediction method and apparatus. The disease prediction apparatus receives a medical image and clinical information, identifies, from the medical image, quantitative data comprising a volume of at least one anatomical structure, and predicts a disease occurrence based on the clinical information and the quantitative data. An artificial intelligence model may be used for each of identification of an anatomical structure and prediction of the disease occurrence.

## Description

The invention relates to a disease prediction method and apparatus.

The disclosure was supported by the "AI Precision Medical Solution (Doctor Answer 2.0) Development" project hosted by Seoul National University Bundang Hospital (Project Serial No.: 1711151151, Project No.: S0252-21-1001). This application is based on and claims priority under 35 U.S.C. §119 to Korean Patent Application No. 10-2022-0134461, filed on October 18, 2022, in the Korean Intellectual Property Office, and Korean Patent Application No. 10-2022-0159560, filed on November 24, 2022, in the Korean Intellectual Property Office, the disclosures of which are incorporated by reference herein in their entireties.

Various diseases (for example, liver cancer, lung cancer, pneumonia, diabetes, etc.) are diagnosed through blood tests, computed tomography (CT), or magnetic resonance imaging (MRI). However, before the occurrence of such a disease, it is possible to simply infer the possibility of occurrence of a specific disease from a patient's eating habits, various underlying diseases, etc. For example, patients with chronic serum hepatitis have a higher chance of developing liver cancer than ordinary people, but it is difficult to accurately predict an actual probability of occurrence of liver cancer for each patient. Moreover, it is difficult to accurately predict whether liver cancer will relapse for a patient treated for liver cancer.

It is the technical problem underlying the invention to provide a disease prediction method and apparatus which alleviate or avoid inconveniencies of prior art disease prediction method and systems and which are capable to predict and provide a probability of disease occurrence before disease occurrence based on images and clinical information.

The invention solves this problem by providing a disease prediction method having the features of claim 1 and a disease prediction apparatus having the features of claim 8. Advantageous embodiments of the invention are mentioned in the dependent claims, the wording of which is herewith incorporated into the description by reference to avoid unnecessary text repetitions. Additional aspects will be set forth in part in the description which follows and, in part, will be apparent from the description, or may be learned by practice of the presented embodiments of the invention.

According to the invention, the disease prediction method includes receiving a medical image and clinical information, identifying, from the medical image, quantitative data including a volume of at least one anatomical structure, and predicting a disease occurrence based on the clinical information and the quantitative data.

According to the invention, the disease prediction apparatus includes a receiving unit configured to receive a medical image and clinical information, a quantitative analyzing unit configured to identify, from the medical image, quantitative data including a volume of at least one anatomical structure, and a predicting unit configured to predict a disease occurrence based on the clinical information and the quantitative data.

The above and other aspects, features, and advantages of the invention will be more apparent from the following description of exemplary embodiments of the invention taken in conjunction with the accompanying drawings, in which:
FIG. 1 is a view showing an example of a disease prediction apparatus according to an embodiment;
FIG. 2 is a flowchart of an example of a disease prediction method according to an embodiment;
FIG. 3 is a view showing an example of a method of separating an anatomical structure through a first artificial intelligence model, according to an embodiment;
FIG. 4 is a view showing an example of a method of predicting a disease through a second artificial intelligence model, according to an embodiment;
FIG. 5 is a view showing an example of an anatomical structure and clinical information used in a disease prediction method, according to an embodiment;
FIG. 6 is a view showing an example of a result screen of separating an anatomical structure through a first artificial intelligence model, according to an embodiment;
FIG. 7 is a view showing an example of a screen of identifying clinical information used in disease prediction according to an embodiment;
FIG. 8 is a view showing an example of an output screen of a disease prediction result according to an embodiment; and
FIG. 9 is a block diagram of an example of a disease prediction apparatus according to an embodiment.

Reference will now be made in detail to embodiments, examples of which are illustrated in the accompanying drawings, wherein like reference numerals refer to like components throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of components, modify the entire list of components and do not modify the individual components of the list.

Hereinafter, a disease prediction method and apparatus according to an embodiment will be described in detail with reference to the accompanying drawings.

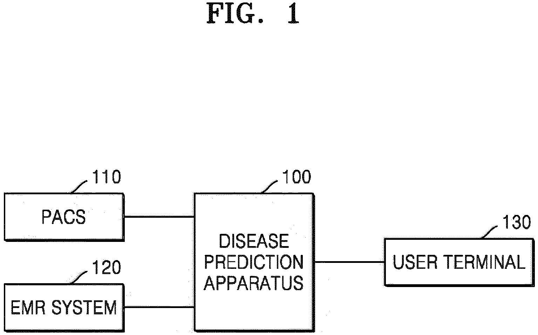

FIG. 1 is a view showing an example of a disease prediction apparatus according to an embodiment.

Referring to FIG. 1, a disease prediction apparatus 100 predicts and outputs a disease occurrence possibility in response to an input of a medical image and clinical information.

In an embodiment, the disease prediction apparatus 100 receives a medical image in a digital image and communication in medicine (DICOM) form from a picture archiving and communication system (PACS)) 110. The medical image may be a three-dimensional (3D) image such as a CT image, an MRI image, etc.

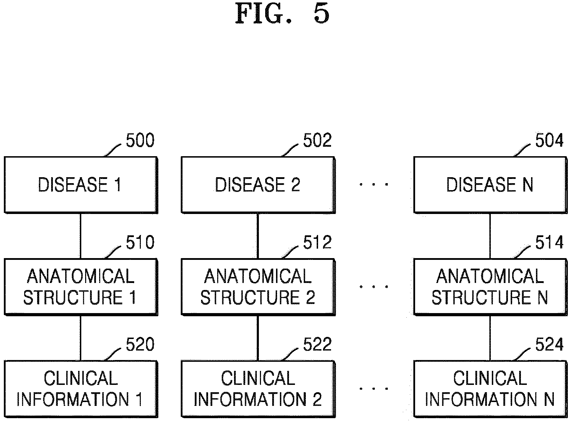

In another embodiment, the medical prediction apparatus 100 receives clinical information from an electronic medical record (EMR) system 120. The clinical information may include various information and/or blood test information, etc., recorded in the EMR. A type of clinical information used for disease prediction may differ with a type of a disease, which will be described again with reference to FIG. 5.

In the current embodiment, an example is shown in which medical images and clinical information are input from the PACS 110 and the EMR system 120, but this is merely an example, and medical images and clinical information may be input through a user terminal 130 or may be directly input from a user through various types of interfaces present in the disease prediction apparatus 100. However, for convenience, a description will be made assuming a case where a medical image and clinical information are input from the PACS 110 and the EMR system 120.

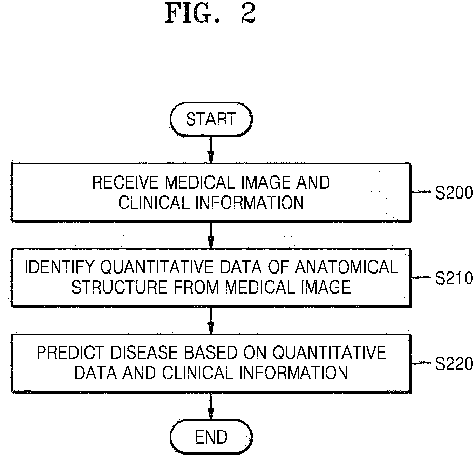

FIG. 2 is a flowchart of an example of a disease prediction method according to an embodiment.

Referring to FIG. 2, the disease prediction apparatus 100 receives a medical image and clinical information of a patient, in operation S200. For example, to predict occurrence of liver cancer of a patient with chronic serum hepatitis or predict a relapse possibility of a patent treated for a curative liver cancer, the disease prediction apparatus 100 may receive a chest CT image and clinical information such as a blood test (e.g., liver cirrhosis, an age, a platelet count, ETV/TDF, a gender, serum ALT, HBV DNA, albumin, bilirubin, HbeAg, etc.) and of the patient.

The disease prediction apparatus 100 identifies quantitative data of the anatomical structure from a medical image, in operation S210. For example, to predict occurrence of liver cancer, etc., the disease prediction apparatus 100 may identify quantitative data including a volume of an anatomical structure such as a liver, a spleen, a muscle, an arteriovenous fistula (AVF), etc. A type of the anatomical structure used for disease prediction may differ with a type of a disease, which will be later described again with reference to FIG. 5.

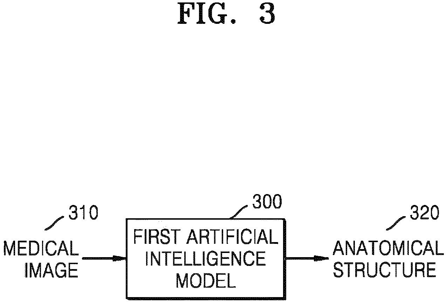

In an embodiment, the disease prediction apparatus 100 separates at least one anatomical structure from a medical image by using a first artificial intelligence model implemented by deep learning, and obtain quantitative data such as a volume, etc., of the separated anatomical structure. A method of identifying the quantitative data of the anatomical structure by using the first artificial intelligence model will be described again with reference to FIG. 3. Moreover, various existing algorithms for separating a human tissue from the medical image may be used, without being limited to the first artificial intelligence model.

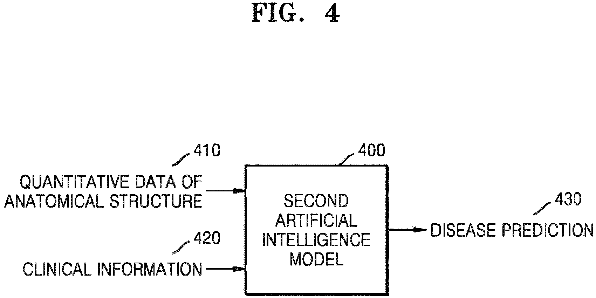

The disease prediction apparatus 100 predicts a disease occurrence possibility based on the quantitative data of the anatomical structure and the clinical information, in operation S220. For example, the disease prediction apparatus 100 may calculate and output a possibility of liver cancer occurrence of a patient with chronic serum hepatitis or a relapse probability of a patient treated for a curative cancer. The disease prediction apparatus 100 may identify a disease occurrence possibility through a second artificial intelligence model implemented by machine learning. A detailed method of identifying the disease occurrence possibility through the second artificial intelligence model will be later described again with reference to FIG. 4.

FIG. 3 is a view showing an example of a method of separating an anatomical structure through a first artificial intelligence model, according to an embodiment.

Referring to FIG. 3, a first artificial intelligence model 300 may be an artificial intelligence model that separates at least one anatomical structure 320 from a medical image 310. For example, the first artificial intelligence model 300 may be implemented with a convolutional neural network (CNN) based on deep learning. The first artificial intelligence model 300 may be implemented with U-Net.

The first artificial intelligence model 300 may be generated through a learning process. The disease prediction apparatus 100 may train the first artificial intelligence model 300 with supervised learning by using learning data including a dataset of the medical image 310 and a separation result (i.e., ground truth). For example, the disease prediction apparatus 100 may input the medical image 310 of the learning data to the first artificial intelligence model 300 to obtain the anatomical structure 320 and train the first artificial intelligence model 300 to reduce a value of a loss function indicating a difference between the anatomical structure 320 and the separation result of the learning data. Training of an artificial intelligence model is already known widely and thus will not be described further.

In an embodiment, the first artificial intelligence model 300 may a model that separates predefined at least one anatomical structure. For example, when a liver cancer occurrence possibility is predicted, the first artificial intelligence model 300 may be a model that separates a 3D liver region from a chest CT image . In another example, the first artificial intelligence model 310 may be a model that separates a plurality of anatomical structures such as a liver region, a spleen region, a muscle region, etc., from a chest CT image.

The disease prediction apparatus 100 may identify quantitative data such as a volume of the anatomical structure 320, etc., separated through the first artificial intelligence model 300. The disease prediction apparatus 100 may identify a volume of an anatomical structure, etc., by applying various existing methods for obtaining a volume of a 3D structure, etc. The current embodiment proposes a volume as an example of quantitative data, but this is merely an example, and various information (e.g., an area, a density, etc.) identifiable from the anatomical structure may be used together as quantitative data.

FIG. 4 is a view showing an example of a method of predicting a disease through a second artificial intelligence model, according to an embodiment.

Referring to FIG. 4, a second artificial intelligence model 400 may be an artificial neural network model to output a disease prediction result 430 in response to an input of quantitative data 410 of an anatomical structure and clinical information 420. For example, the second artificial intelligence model 400 may be implemented with a gradient boosting machine (GBM) based on machine learning.

The second artificial intelligence model 400 may be generated through a learning process. The disease prediction apparatus 100 may train the second artificial intelligence model 400 by using learning data including a dataset of the quantitative data 410 of the anatomical structure, the clinical information 420, and information indicating whether a disease breaks out. For example, the disease prediction apparatus 100 may train the second artificial intelligence model 400 based on a loss information indicating a difference between information, of the learning data, indicating whether the disease breaks out and a disease prediction result 430 obtained by inputting the quantitative data 410 and the clinical information 420 of the learning data to the second artificial intelligence model 400. For example, the loss function may be defined as binary logistic regression. A method of training an artificial neural network such as GBM, etc., is already known and thus will not be described in detail.

FIG. 5 is a view showing an example of an anatomical structure and clinical information used in a disease prediction method, according to an embodiment.

Referring to FIG. 5, the disease prediction apparatus 100 may predict an occurrence possibility (i.e., an occurrence/relapse probability, etc.) of at least one diseases 500, 502, and 504. As an anatomical structure and/or clinical information used to predict each disease 500, 502, or 504 may be different, the disease prediction apparatus 100 may predefine and store a mapping relationship between an anatomical structure and clinical information for each disease.

In an embodiment, the disease prediction apparatus 100 may store a first anatomical structure 510 and first clinical information 520 by mapping them to a first disease 500, store a second anatomical structure 512 and second clinical information 522 by mapping them to a second disease 502, and store an Nth anatomical structure 514 and Nth clinical information 524 by mapping them to an Nth disease 504. Some or all of the first to Nth anatomical structures 510, 512, and 514 may be identical to or different from one another. Some or all of the first to Nth clinical information 520, 522, and 524 may be identical to or different from one another.

For example, when the first disease 500 is liver cancer, the first anatomical structure 510 may include a liver, a spleen, and muscle, and the first clinical information 520 may include age, gender, antiviral drug use (ETV or TDF), liver cirrhosis, blood albumin concentration, blood bilirubin level, blood alanine aminotransferase concentration, HBV DNA level, whether serum hepatitis virus is positive for an envelope antigen, etc.

The optimal anatomical structures 510, 512, and 514 and the optimal clinical information 520, 522, and 524 respectively used for prediction of occurrence possibilities of the diseases 500, 502, and 504 may be identified in advance through an experiment, etc., and defined as shown in FIG. 5. In an embodiment, the anatomical structures 510, 512, and 514 and the clinical information 520, 522, and 524 may be updated by a future clinical test result, etc., and an anatomical structure and clinical information for new disease prediction may be added. To this end, the disease prediction apparatus 100 may provide a user interface for receiving a type of a disease, and a type of an anatomical structure and a type of clinical information, which are suitable for prediction of the disease, from a user.

Upon receiving a prediction request for a second disease 502 of a patient from a user, the disease prediction apparatus 100 may receive a medical image of the patient from the PACS 110, separate a human body tissue defined in the second anatomical structure 512 from the medical image, and identify quantitative data. The disease prediction apparatus 100 may receive information corresponding to second clinical information 522 from the EMR system 120, etc.

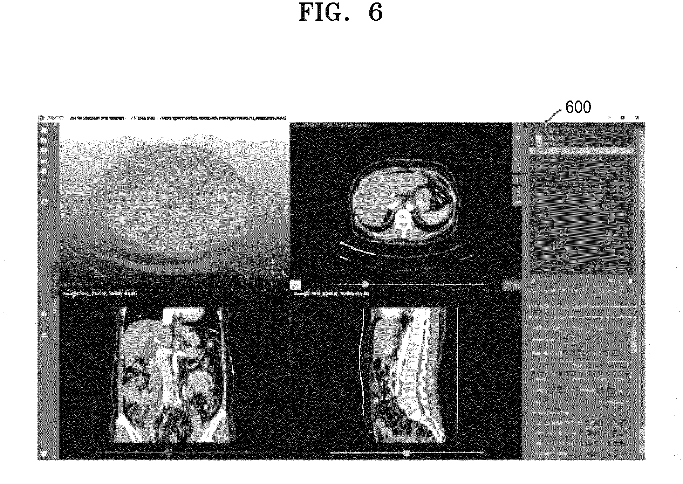

FIG. 6 is a view showing an example of a result screen of dividing an anatomical structure through a first artificial intelligence model, according to an embodiment.

Referring to FIG. 6, the disease prediction apparatus 100 may display an anatomical structure separated from the medical image through the first artificial intelligence model on a screen 600. For example, the disease prediction apparatus 100 may display sagittal, coronal, and axial images of the anatomical structure or display a 3D modeling result.

In another example, when the plurality of anatomical structures are separated from the medical image, the disease prediction apparatus 100 may display the plurality of anatomical structures in different colors or textures, etc., together. Alternatively, the disease prediction apparatus 100 may provide a screen interface through which the user may select any one of the plurality of anatomical structures, and when the user selects a specific anatomical structure through the screen interface, the disease prediction apparatus 10 may display the selected anatomical structure on the screen.

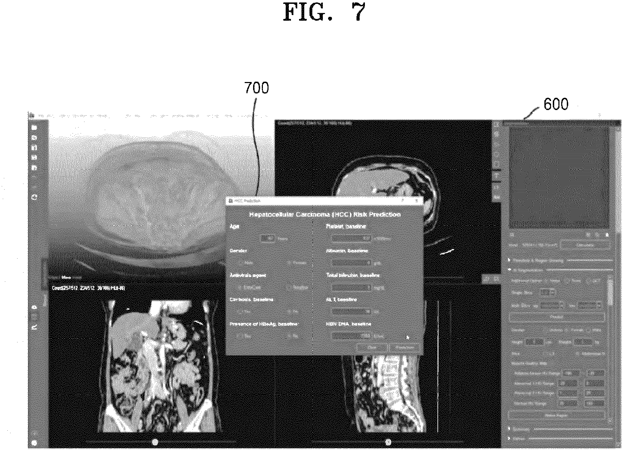

FIG. 7 is a view showing an example of a screen of identifying clinical information used in disease prediction according to an embodiment.

Referring to FIG. 7, the disease prediction apparatus 100 may receive clinical information required for disease prediction from the EMR system 120 and display the clinical information on a screen interface 700. The disease prediction apparatus 100 may enable the user to modify the clinical information displayed on the screen interface 700. In another example, the disease prediction apparatus 100 may directly receive the clinical information from the user through the screen interface 700.

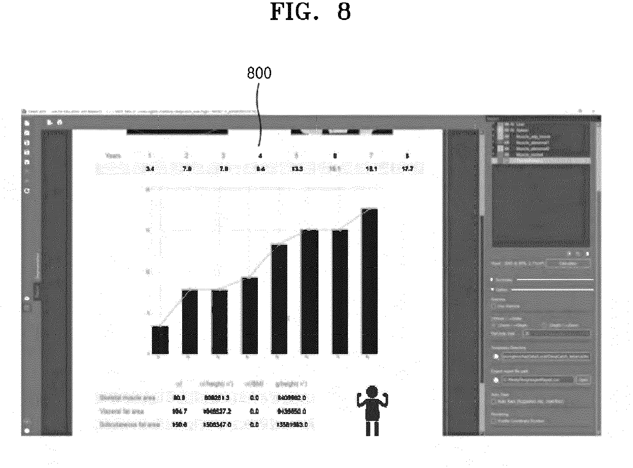

FIG. 8 is a view showing an example of an output screen of a disease prediction result according to an embodiment.

Referring to FIG. 8, the disease prediction apparatus 100 may output a disease occurrence possibility in the form of a graph 800, etc. For example, the disease prediction apparatus 100 may generate a disease occurrence probability over time in the form of a visual material such as a table, a graph, etc., and transmit the same to a user terminal, etc.

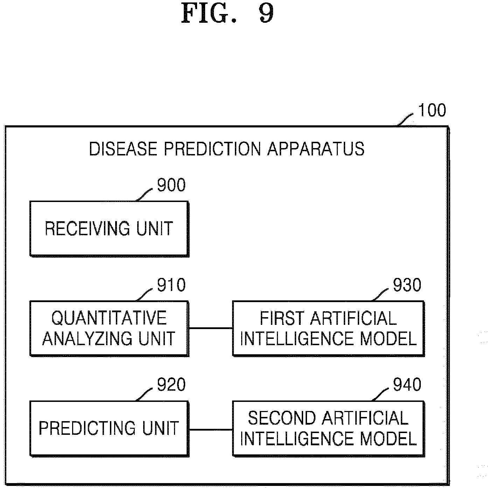

FIG. 9 is a block diagram of an example of a disease prediction apparatus according to an embodiment.

Referring to FIG. 9, the disease prediction apparatus 100 includes a receiving unit 900, a quantitative analyzing unit 910, a predicting unit 920, a first artificial intelligence model 930, and a second artificial intelligence model 940. In an embodiment, the disease prediction apparatus 100 may be implemented as a computing device including a memory, a processor, and an input/output device. In this case, each component may be implemented with software and loaded on the memory, and then may be executed by the processor.

The receiving unit 900 may receive a medical image and clinical information. For example, the receiving unit 900 may receive a medical image and clinical information from a PACS, an EMR system, etc. In another embodiment, when various diseases are predictable as shown in FIG. 5, the receiving unit 900 may request an anatomical structure and clinical information required for prediction of a disease to the PACS and the EMR system and receive them from the PACS and the EMR system.

The quantitative analyzing unit 910 may separate an anatomical structure from a medical image by using the first artificial intelligence model 930 and identify quantitative data such as a volume of the separated anatomical structure, etc. In an embodiment, the first artificial intelligence model 930 may a model that separates an anatomical structure of a specific type. In this case, there may be a plurality of first artificial intelligence models that separate anatomical structures of different types. For example, a first-first artificial intelligence model may separate a liver region, and a first-second artificial intelligence model may separate a spleen region. In another embodiment, the first artificial intelligence model 930 may be a model that separates any one of the plurality of anatomical structures.

The predicting unit 920 may input quantitative data of an anatomical structure and clinical information to the second artificial intelligence model 940 to predict a disease occurrence probability. An example of the second artificial intelligence model 940 is shown in FIG. 4.

The invention may also be implemented as a computer-readable program code on a computer-readable recording medium. The computer-readable recording medium may include all types of recording devices in which data that is readable by a computer system is stored. Examples of the computer-readable recording medium may include read-only memory (ROM), random access memory (RAM), compact-disc ROM (CD-ROM), a magnetic tape, a floppy disk, an optical data storage device, etc. The computer-readable recording medium may be distributed over computer systems connected through a network to store and execute a computer-readable code in a distributed manner.

So far, embodiments have been described for the invention. It would be understood by those of ordinary skill in the art that the invention may be implemented in a modified form within a scope without departing from the essential characteristics of the invention. Therefore, the disclosed embodiments should be considered in a descriptive sense rather than a restrictive sense. The scope of the present specification is not described above, but in the claims, and all the differences in a range equivalent thereto should be interpreted as being included in the invention.

According to an embodiment, the accuracy of disease occurrence prediction may be improved using a medical image and clinical information together. For example, a liver cancer occurrence possibility may be predicted and provided to a patient with chronic serum hepatitis, or a relapse possibility may be predicted and provided to a patient treated for liver cancer.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the spirit and scope of the disclosure as defined by the following claims.

## Claims

1. Disease prediction method comprising:
receiving a medical image and clinical information;
identifying, from the medical image, quantitative data comprising a volume of at least one anatomical structure; and
predicting a disease occurrence based on the clinical information and the quantitative data.

2. Disease prediction method of claim 1, wherein the medical image is a computed tomography (CT) image.

3. Disease prediction method of claim 1 or 2, wherein the clinical information comprises an electronic medical record and/or blood test information.

4. Disease prediction method of any of claims 1 to 3, wherein the identifying of the quantitative data comprises:
separating the at least one anatomical structure from the medical image by using a first artificial intelligence model; and
identifying the volume of the at least one anatomical structure.

5. Disease prediction method of any of claims 1 to 4, wherein the predicting of the disease occurrence comprises inputting the clinical information and the quantitative data to a second artificial intelligence model configured to output a disease prediction value.

6. Disease prediction method of any of claims 1 to 5, wherein the receiving comprises receiving clinical information of a predefined type according to a type of a disease.

7. Disease prediction method of any of claims 1 to 6, wherein the receiving comprises:
receiving the medical image from a picture archiving and communication system (PACS); and
receiving the clinical information from an electronic medical record system (EMS).

8. Disease prediction apparatus comprising:
a receiving unit (900) configured to receive a medical image and clinical information;
a quantitative analyzing unit (910) configured to identify, from the medical image, quantitative data comprising a volume of at least one anatomical structure; and
a predicting unit (920) configured to predict a disease occurrence based on the clinical information and the quantitative data.

9. Disease prediction apparatus of claim 8, further comprising:
a first artificial intelligence model (930) configured to separate the at least one anatomical structure from the medical image; and
a second artificial intelligence model (940) configured to output a disease prediction value in response to an input of clinical information and quantitative data thereto,
wherein the quantitative analyzing unit is further configured to identify the quantitative data by using the first artificial intelligence model, and
the predicting unit is further configured to predict the disease occurrence by using the second artificial intelligence model.

10. Disease prediction apparatus of claim 9, wherein the first artificial intelligence model is a convolutional neural network (CNN) model, and the second artificial intelligence model is a gradient boosting machine (GMB) model.

11. Computer-readable recording medium having recorded thereon a computer program for executing the disease prediction method of any of claims 1 to 7.
